# EUROPEAN PATENT SPECIFICATION

(11) **EP 2 263 668 B1**
(45) Date of publication and mention of the grant of the patent: **14.05.2014**
(21) Application number: 09716802.5
(22) Date of filing: 05.03.2009
(51) Int. Cl.: A61P 35/02

(54) **USE OF TEA POLYPHENOLS IN PREPARING MEDICAMENTS FOR PREVENTION OR TREATMENT OF TUMORS**
VERWENDUNG VON TEE-POLYPHENOLEN BEI DER HERSTELLUNG VON MEDIKAMENTEN ZUR PRÄVENTION ODER BEHANDLUNG VON TUMOREN
UTILISATION DE POLYPHÉNOLS DE THÉ DANS LA PRÉPARATION DE MÉDICAMENTS DESTINÉS À LA PRÉVENTION OU AU TRAITEMENT DE TUMEURS

(30) Priority: 06.03.2008 CN 200810034350
(43) Date of publication of application: 22.12.2010
(73) Proprietor: Zhang, Li, Shanghai 200023 (CN); Liu, Yunhua, Shanghai 200052 (TW)
(72) Inventor: Zhang, Li, Shanghai 200023 (CN); Liu, Yunhua, Shanghai 200052 (TW)
(74) Representative: Campbell, Neil Boyd
(86) International application number: PCT/CN2009/000233
(87) International publication number: WO 2009/109109

(56) References cited:
- WO-A1-01/72318
- WO-A1-01/72319
- US-A1- 2005 031 716
- DATABASE WPI Week 200527 Thomson Scientific, London, GB; AN 2005-256986 XP002672318, & JP 2005 075790 A (FUJII N) 24 March 2005 (2005-03-24)
- DATABASE WPI Week 200174 Thomson Scientific, London, GB; AN 2001-642014 XP002672319, & JP 2001 226276 A (KEIZAI SANGYOSHO SANGYO GIJUTSU SOGO KEN) 21 August 2001 (2001-08-21)
- DATABASE EMBASE [Online] ZHAO-Y ET AL: 'Apoptosis induced by tea polyphenols in HL,-60 cells', XP008143471 Database accession no. 1998004159 & ZHAO-Y ET AL.: 'Apoptosis induced by tea polyphenols in HL,-60 cells.' CANCER-LETT. vol. 121, no. 2, 1997, pages 163 - 167
- LI BIN ET AL.: 'Effect of the Tea Polyphenols (TP) on Growth Inhibition of Leukemia Cell Line K562 in Vitro.' JOURNAL OF JINZHOU MEDICAL COLLEGE. vol. 27, no. 2, 2006, pages 19 - 21, XP008145742

## Description

### Technical Field

The invention is related to the use of tea polyphenols or pharmaceutically acceptable salts thereof or any pharmaceutical compositions containing any one of them in preparing medicaments for prevention or treatment of tumors.

### Background of the Invention

Nowadays, the methods for medical treatment of tumors mainly include cytotoxin, induction of apoptosis and differentiation, biological targeted therapy, etc.

Some components originated from natural plants and minerals, such as leurocristine, homoharringtonine, hydroxycamptothecine, paclitaxel and arsenious acid, etc., play a significant role in the first two methods. However, the toxic and side effects, such as the inhibition of proliferation and growth of normal cells in varying degrees, the toxic effects on the peripheral nervous system and the damage to the vital organs, are the important factors which restrict the use of these drugs, and the pharmacoeconomics is also a major factor which cannot be ignored.

The biological targeted therapy uses medicaments which are "custom-made" according to the pathogenesis of tumors including Imatinib mesylate (Imatinib, a tyrosine kinase inhibitor aimed at chronic granulocytic leukemia), rituximab, etc. The price of the medicaments is a large obstacle to the widespread use of those medicaments, and the drug resistance caused by target mutation is a fatal disadvantage.

Because of the above factors, at present, medicaments for treatment of tumors are often used in combination. However, the median lethal dose (LD50) of some medicaments is close to the effective concentration, which easily causes toxic and side effects such as distinct myelosuppression, damage to vital organs, etc, and the tolerance is poor. Also the toxic and side effects of various medicaments have synergistic effects. Therefore the therapeutic effects are only enhanced to some extent, and still a considerable number of patients have drug resistance and show refractoriness and relapse. The limited therapeutic effects of some high bio-technology products are not proportional to their high price. In addition, those products have high preservation requirements and are inconvenient to use. All these factors are major reasons that prompt human beings to keep exploring for new components and new methods to treat tumors.

Tea leaves from Chinese common tea plants are rich in nutriments such as Vitamin C, chlorophyll, carotene, tea polyphenols, etc. Tea polyphenols include catechins, which include epigallocatechin gallate (EGCG), epigallocatechin (EGC), epicatechin gallate (ECG), and epicatechin (EC). Among them, EGCG is the most abundant one and has the most extensive biological activity, but EGC has stronger anti-oxidant effects than EGCG. Other strong epidemiological evidences also show that an increase in internal concentration of tea polyphenols can provide a powerful protection, eliminate the damage of oxygen free radicals to the body, and have effects on prevention of cancers. Drinking tea has antitoxic and sterilization effects, prevents cancer, and prolongs life. Its healthful effects are well known, but it has not yet been further verified that to what extent and in which aspects the benefits can be.

Further, because catechins in many tea plants are different in their components and contents, and the production process is not standardized, there are at present a variety of edible catechin products and their content and quality are uncertain. They generally belong to the category of health products. The research abroad in inhibiting tumor proliferation using EGCG is mainly in vitro, and the studied cell lines include solid tumor cells such as prostatic cancer, gastric cancer, colon carcinoma, skin cancer, breast cancer, lung cancer, leiomyosarcoma etc. However, its activity of inhibiting tumor proliferation is not very strong and pure EGCG and EGC products are very expensive. Therefore, there is no further report of the in vivo research.

### Summary of the Invention

In view of the above defects of the prior art, the object of the invention is to provide medicaments for treatment of tumors which are low-cost and convenient to use, and have nearly no toxic and side effects.

The present invention is mainly based on such an idea: although research indicates that catechins like epigallocatechin gallate (EGCG) have anti-tumor effects, the price is very high to obtain various pure catechins products through purification of tea polyphenols from plant extracts. If tea polyphenols, which are unpurified and are rich in catechins such as EGCG etc., can be used directly as antitumor medicaments, the production cost will be greatly reduced.

Therefore, the present invention provides uses of tea polyphenols or pharmaceutically acceptable salts thereof or pharmaceutical compositions containing any one of them in preparing medicaments for prevention or treatment of tumors.

The tea polyphenols are tea leave extracts, which include catechins mainly consisting of epigallocatechin gallate (EGCG), epicatechin gallate (ECG) and epigallocatechin (EGC).

In an embodiment of the invention, the pharmaceutical formulations of the medicaments can be any pharmaceutically acceptable formulations such as tablets, capsule, buccal tablets, orally disintegrating tablets, oral fast dissolving tablets, dispersible tablet, masticatory, granules, dry suspension, injection, solution, slow-release formulation, controlled-release formulation, rapid-release formulation, etc.

In an embodiment of the invention, the pharmaceutical compositions also include pharmaceutically acceptable excipients or carriers.

In a preferred embodiment of the invention, the tannin contained in tea leaves is removed from the tea polyphenols, and caffeine, which is an undesirable component, is less than 0.2% by weight in the made formulation.

In an embodiment of the invention, EGCG, ECG and EGC in the tea polyphenols are 50-60 %, 20∼25% and 8∼10 % by weight of the catechins, respectively.

In an embodiment of the invention, the capsule is Tegreen97^{®}, a green tea capsule of Pharmnex INC. USA.

The formulation in the invention can be used alone to treat tumors without a combination with other medicaments.

The invention utilizes tea polyphenols, which are tea leave extracts, to prepare antitumor formulations directly instead of pure products of the active components such as EGCG, ECG and EGC etc. separated from tea polyphenols through complicated and costly purification processes. Thus the production cost of the formulations may be greatly reduced.

Moreover, it is discovered through pharmacological tests in cellular level that tea polyphenols involved in the invention have significant inhibition effects on many kinds of human tumor cells, especially malignant tumor cells in hematopoietic system, and even show good inhibition effects on primary culture cells in some blood tumors. This not only adds a new drug of high-efficiency and low-toxicity for treatment of malignant tumors, but also provides the patients, who suffer from secondary vasculitis after a long-term intravenous infusion of cytotoxin medicaments, with a possibility of keeping being treated in other ways. Through the efficacy test of tumor models in mice, the formulations involved in the invention have significant therapeutical effects on mice tumors. They can reduce the size of the tumors and prolong the survival period of the mice bearing tumor models. Furthermore, the tea polyphenols can be used alone to achieve the above pharmaceutical effects substantially without any other adjuvant therapy for the symptoms.

Moreover, the tea polyphenols involved in this invention hardly have any toxic and side effects of the above-mentioned existing medicaments have. In addition, the plant source of the tea polyphenols is widely spread, and with an abundant supply of the raw materials, and the economic benefit is obvious.

Moreover, the formulations can be capsule, which is very convenient to use and may avoid the damage and stimulation to the tissue and blood vessels caused by injection formulation cytotoxin medicaments which are commonly used before.

Besides, though the tea polyphenols are extracted from the plant, the standardization of the production process can keep the contents of main components of the extracts stable and prevent the difference in contents of components in different batches. Thereby the corresponding research will have good repeatability and comparability. It is a promising product for treatment of tumors. As the formulations for treatment of tumors made of the tea polyphenols do not synergistically increase toxic and side effects when used in combination with other medicaments, and especially have no inhibiting effects on the normal hematopoiesis of the bone marrow, it also provides a promising medicament which can be combined with other medicaments to increase the therapeutic effects together.

Hereinafter, the technical effects of this invention will be further demonstrated with reference to the drawings in order to fully understand the invention.

### Description of the Drawings

Fig. 1 is a graph representing proliferation inhibiting effects of tea polyphenols solutions of varying concentrations on a variety of cell lines for 1 day.
Fig. 2 is a graph representing proliferation inhibiting effects of 129.6µmol/L solution on a variety of cell lines for 1∼3 days.
Fig. 3 shows photos of cell morphology observed by a light microscope, representing the effects of tea polyphenols solutions of varying concentrations on morphology of RPMI8226 cell line for 1 day.
Fig. 4a∼Fig. 4c are scatter diagrams representing the effects of solutions of varying concentrations on the apoptosis rate of RPMI8226 cell line for 1 day.
Fig. 5a Fig. 5c are scatter diagrams representing the effects of solutions of varying concentrations on the apoptosis rate of U937 cell line for 2.5 days.
Fig. 6a Fig. 6d are peak diagrams representing the effects of solutions of varying concentrations on the cell cycle of RPMI8226 cell line for 2 days.
Fig. 7 is a scatter diagram representing the effects of solutions of varying concentrations on the mitochondrial membrane potential of RPMI8226 cell line for different duration.
Fig. 8 shows photos of cell morphology observed by a light microscope, representing the effects of solutions of varying concentrations on morphology of ALL-L2 primary culture cell for 1.5 days.
Fig. 9 is a scatter diagram representing the effects of solutions of varying concentrations on the apoptosis rate of ALL-L2 primary culture cell for 1.5 days.
Fig. 10 shows cell morphology photos of mice tumor tissue sections observed by a microscope.

### Detailed Description of the Embodiments

The present invention provides a new use of natural tea polyphenols extracted from plants as antitumor formulations. Tea polyphenols involved in the invention are tea leave extracts and are rich in catechins, which includes epigallocatechin gallate (EGCG), epicatechin gallate (ECG), and epigallocatechin (EGC).

The antitumor formulations made of tea polyphenols can be any pharmaceutically acceptable formulations such as tablets, capsule, buccal tablets, orally disintegrating tablets, oral fast dissolving tablets, dispersible tablet, masticatory, granules, dry suspension, injection, solution, slow-release formulation, controlled-release formulation and rapid-release formulation.

Although the change of content of catechins in tea polyphenols does not affect its antitumor effects significantly, generally, in order to ensure the stability of every batch of products and the comparability of effects, process, in the embodiments of the invention, through the control over the production, the content of catechins is controlled at 60∼70 % by weight in the antitumor solid formulations made of tea polypheols.

In addition, catechins mainly consist of EGCG, ECG, and EGC. They all have certain antitumor effects. Therefore there are no special restrictions on the proportions of EGCG, ECG and EGC with respect to one another in the tea polyphenols of the invention. Usually, EGCG is the main component among the three. For instance, it contributes more than 50 percent of total weight of the three. EGC is the least. For instance, it contributes about 10 percent or less. However, in order to ensure the stability of every batch of the products and comparability of the effects, through control over production process, EGCG, ECG and EGC are controlled at 50∼60%, 20∼25 % and 8∼10 % respectively of the total weight of the three in the tea polyphenols of the invention or formulations made of it for treatment of tumors.

To increase the applicability of the formulations made of tea ployphenols described above, during the production of tea polyphenols, undesirable components like tannin and caffeine in the tea leaves can be removed. Moreover, it is better to ensure the content of caffeine is less than 0.5mg in every 250mg of the final formulations for treatment of tumors. Almost all people can tolerate such a caffeine level.

The tea polyphenols used in the invention can be made into a variety of pharmaceutical formulations by common pharmaceutical methods, for instance, powder, tablets, capsules or solutions. When in use, the formulations can be taken orally directly or used after dissolving in water.

This mixture formulation of tea polyphenols can well inhibit the proliferation of various tumor cells in vitro. The mechanism may be induction of cell apoptosis through the way of caspase-3, etc. In addition, compared with pure EGCG products and some cytotoxic medicaments, its proliferation inhibition effects are only better. This mixture formulation even has good inhibition effects on some tumor primary culture cells. Taking this tea polyphenols formulation orally can reach the effective antitumor concentration, and more effectively inhibit growth of human multiple myeloma RPMI8226 cell, Lymphoma Jurkat cell, Sudhl-4 cell, etc., inside nude mice.

### Embodiments

The source of experimental material
1. Cell line NB4, NB4-R1, NB4-R2, U937, K562 and RPMI8226 are donated by Ruijin Hospital, School of Medicine of Shanghai Jiaotong University, and Shanghai Institute of Hematology.
   Names of the tumor cell lines used herein:
   NB4: Human acute promyelocytic Leukemia cell line
   NB4-R1: Resistant to all-trans retinoic acid human acute promyelocytic Leukemia cell line 1
   NB4-R2: Resistant to all-trans retinoic acid human acute promyelocytic Leukemia cell line 2
   U937: Human acute monocytic leukemia cell line
   K562: Human chronic myelocytic Leukemia cell line
   RPMI8226: Human multiple myeloma cell line
2. Experimental mice and nude mice: through Shanghai Jiaotong University, School of Medicine, department of laboratory animal science, purchased from Shanghai SLAC laboratory animal INC. (SCXK (HU) 2003-0003).
3. The formulation for treatment of tumors is made of tea polyphenols from green tea extracts, and the formulation is Tegreen97^{®}, a green tea capsule of Pharmanex INC. USA. There is powder of tea polyphenol in the capsule. The total weight of each capsule is 250mg, among which catechins are 162 mg (containing 95mg of EGCG, 37mg of ECG and 15mg of EGC, with a molecular weight of 458.4, 442.4 and 306.3, respectively) and the content of caffeine is less than 0.5mg. (For more detailed components, referring to: Physicians' desk reference^{®}: Ingredients of Tegreen97^{®}. Pharmanex, 2005, 59Edition:2782.)

The dosage mentioned below is determined by the content of EGCG in Tegreen97^{®}, a green tea capsule, as a reference. Dose of 32.4µmol/L, 64.8µmol/L, 129.6µmol/L and 259.2µmol/L are respectively equivalent to the supernatant concentration obtained after diluting contents of one capsule by 1:6400, 1:3200, 1:1600, 1:800.

Tea polyphenols solution prepared by Tegreen97^{®}, a green tea capsule, underwent the following antitumor tests:

First, the effects on NB4, NB4-R1, NB4-R2, U937, K562, and RPMI8226 cell lines:
1) Cell growth inhibition test: cells survival rate measured by MTT
2) Wright's stain: observing the morphology change of cell
3) Annexin V-FITC and PI double labeling: quantitative detection of apoptosis rate
4) Cell cycle and analysis of DNA concentration in cell nucleus: revealing possible mechanism of induction of apoptosis
5) Analysis of mitochondrial membrane potential: revealing possible mechanism of induction of apoptosis

Second, the effects on primary culture cell of patients of acute lymphoblastic leukemia and acute monocytic leukemia:
1) Wright's stain: observing the morphology change of the cell
2) Annexin V-FITC and PI double labeling: quantitative detection of apoptosis rate

Third, the therapeutic effects of tea polyphenols solution prepared by Tegreen97®, a green tea capsule, on tumor model in nude mice:

The above tests and results are described in detail as follows.

First, the effects on NB4, NB4-R1, NB4-R2, U937, K562, and RPMI8226 cell lines.

### 1) Cell growth inhibition test: cell survival rate measured by MTT

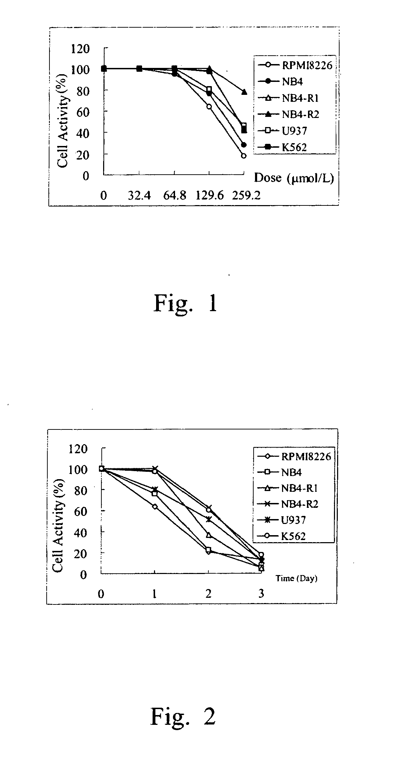

The curve in Fig. 1 shows proliferation inhibiting effects of tea polyphenols of Tegreen97^{®} capsule in the dosages of 32.4µmol/L, 64.8µmol/L, 129.6µmol/L, and 259.2µmol/L on a variety of cell lines for the duration of 1 day.

The curve in Fig. 2 shows proliferation inhibiting effects of 129.6mol/L dosage on a variety of cell lines for the duration of 1∼3 days.

The results indicate that there are significant proliferation inhibition effects on each cell line in the dosage of 129.6 and 259.2mol/L for the duration of 2∼3 days.

### 2) Wright's stain: observing the morphology change of cell apoptosis

Through Wright's stain, the morphology change of cell apoptosis is observed by the light microscope at 100 times.

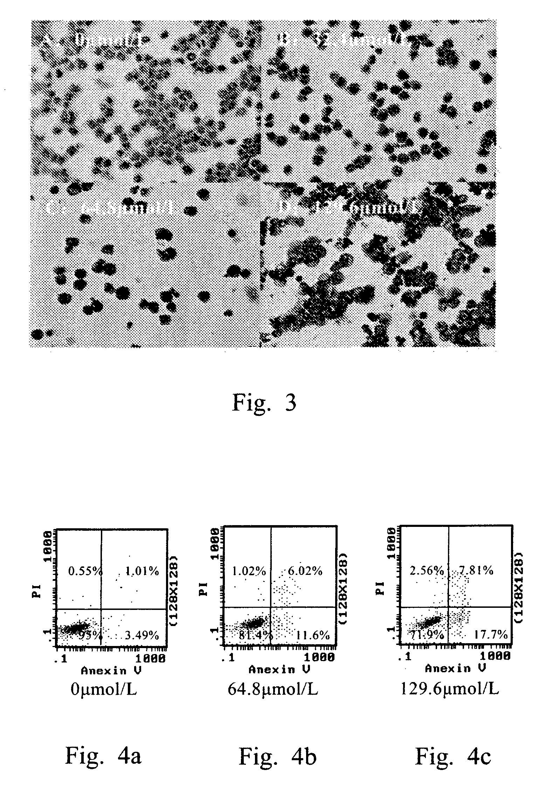

As shown in Fig.3, after the tea polyphenols solution of varying concentrations acted on multiple myeloma cell line RPMI8226 for 1 day, the control group (0µmol/L) shows evenly lightly stained cell nuclei, loose chromatin and normal proportion of cytoplasmic; after 32.4µmol/L, 64.8µmol/L, 129.6µmol/L, and 259.2µmo/L of tea polyphenols acted on RPMI8226 cell for 1 day, membrane shrinkage, chromatin condensation, pyknotic nucleus and apoptosis body appeared.

The results indicate tea polyphenols can induce apoptosis of RPMI8226 cell line, and the higher the concentration is, the more obvious the effects are.

### 3) Annexin V-FITC and PI double labeling: quantitative detection of apoptosis rate

### Multiple myeloma cell line RPMI8226

### Quantitative detection of apoptosis rate by flow cytometry (Annexin V-FITC and PI double labeling method)

FITC, Annexin-V labeled by fluorescein isothiocyannate, combined with externalized membrane phosphatidylserine (PS) when apoptosis happened. Apoptotic cells showed Annexin-V positive.

PI, propidium iodide, combined with nucleic acid. Apoptotic cells showed PI positive.

Fig. 4a ∼ Fig. 4c are fluorescence scatter diagrams indicated by flow cytometry. In the diagrams, the lower left quadrant shows Annexin-V FITC and PI double-labeled negative cells (normal cells); the lower right quadrant shows Annexin-V FITC single-labeled positive cells (early apoptotic cells); the upper right quadrant shows Annexin-V FITC and PI double-labeled positive cells (late apoptotic cells); the upper left quadrant shows PI single- labeled positive cells (dead cells).

The results indicate that, after tea polyphenols of varying concentrations acted on multiple myeloma cell line RPMI8226 for 1 day, in control group (0µmol/L) normal cells are 95%. After tea polyphenols of concentrations of 64.8µmol/L and 129.6µmol/L acted on RPMI8226 cell line for 1 day, early apoptotic and late apoptotic cells increased obviously.

### Human acute monocytic leukemia cell line U937

### Quantitative detection of apoptosis rate by flow cytometry (Annexin V-FITC and PI double labeling method)

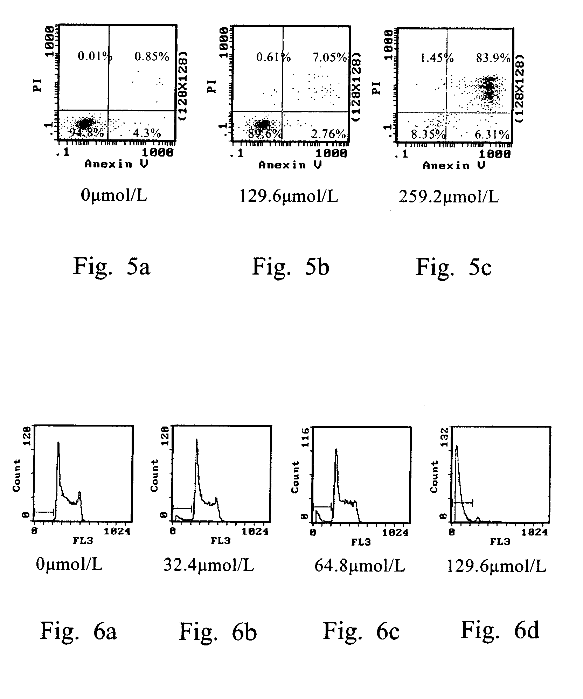

Fig. 5a ∼ Fig. 5c are fluorescence scatter diagrams indicated by the flow cytometry. In the diagrams, the lower left quadrant shows Annexin-V FITC and PI double-labeled negative cells (normal cells); the lower right quadrant shows Annexin-V FITC single-labeled positive cells (early apoptotic cells); the upper right quadrant shows Annexin-V FITC and PI double-labeled positive cells (late apoptotic cells); the upper left quadrant shows PI single- labeled positive cells (dead cells).

The results indicate that, after tea polyphenols of varying concentrations acted on human acute monocytic leukemia cell line U937 for 2.5 days, in control group (0µmol/L) normal cells are 94.7%. After tea polyphenols of concentrations of 129.6 µmol/L and 259.2 µmol/L acted on RPMI8226 cell line for 2.5 days, early apoptotic and late apoptotic cells increased obviously.

### 4) Cell cycle and analysis of DNA concentration in nucleus: revealing possible mechanism of induction of apoptosis

Measured by the flow cytometry, after solutions of varying concentration acted on human multiple myeloma cell line RPMI8226 for 2 days, there was no significant difference in the proportions in the G1, S, G2 stages (which are 32%, 64.4% and 3.5%, respectively).

Fig. 6a ∼ Fig. 6d are peak-shaped diagrams indicated by the flow cytometry. In the diagrams, FL3 and count represent the percentage of hypodiploid cells, which are an indicator of apoptosis. By analysis of the DNA content in the nucleus, with the increase of the concentration, the percentage of hypodiploid cells also increased (in control group, after tea polyphenols of concentration of 32.4µmol/L, 64.8µmol/L and 129.6µmol/L acting for 2 days, the percentages of hypodiploid cells were 0.5 %, 8.3 %,15.7 %and 94.1 %, respectively).

The results indicate that tea polyphenols inhibit the proliferation of human multiple myeloma cell line by the means of inducing apoptosis, which correlates to the concentrations.

### 5) Analysis of mitochondrial membrane potential: revealing possible mechanism of induction of apoptosis

By means of the flow cytometry (Rhodamine Rh123 fluorescent labeling method), mitochondrial membrane potentials inside the cells were quantitatively detected to explore possible mechanism of proliferation inhibition.

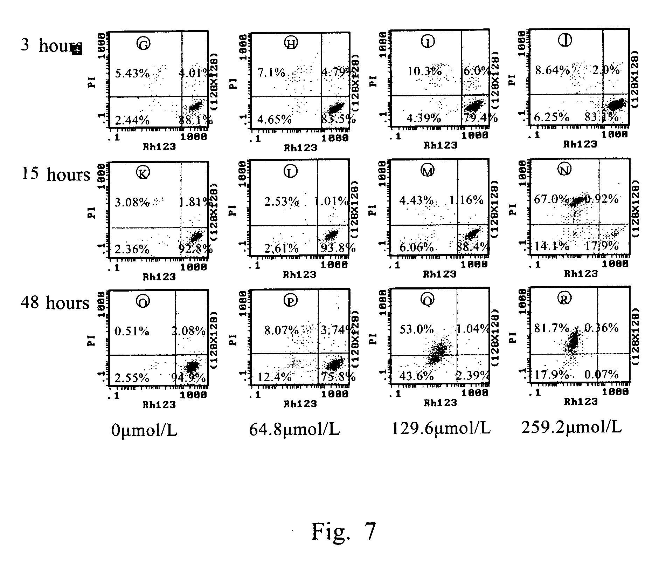

G - R in Fig. 7 provide conditions about the proliferation and apoptosis of human multiple myeloma cell line under different dosages and different acting time periods. In comparison with the control group (0µmol/L), after 64.8µmol/L, 129.6µmol/L, and 259.2µmol/L tea polyphenols acted on human multiple myeloma cell line for 3 hours, mitochondrial membrane potential began to decline and was in a time-dependant manner.

The results indicate that the mechanism of tea polyphenols for inhibiting the proliferation of human multiple myeloma cell line is relevant to induction of apoptosis and may be relevant to the damage on mitochondria.

Second, the effects on primary culture cells of patients of acute lymphoblastic leukemia and acute monocytic leukemia:

### 1) Wright's stain: observing morphology change of cells

Through Wright's stain, morphology change of cells is observed.

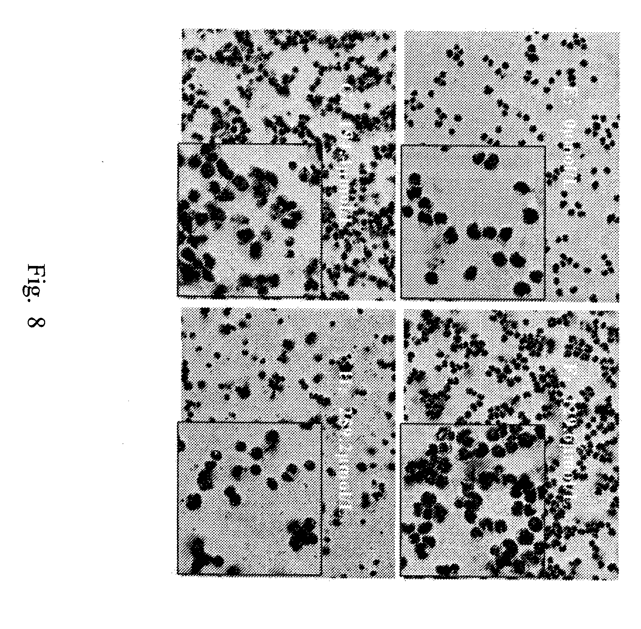

As shown in Fig. 8, after tea polyphenols of varying concentrations acted on primary culture cells of patients of acute lymphoblastic leukemia for 1.5 days, the control group (0µmol/L) showed evenly lightly stained cell nuclei, loose chromatin and normal proportion of cytoplasmic; after tea polyphenols of concentrations of 129.6µml/L, 194.4µmol/L and 259.2µmol/L acting for 1.5 days, membrane shrinkage, chromatin condensation, pyknotic nucleus and eosinophilic body (apoptosis body) appeared in primary culture cells.

The results indicate that tea polyphenols can induce apoptosis of primary culture cells of patients of acute lymphoblastic leukemia, and the higher the concentration is, the more obvious the effects are.

### 2) Annexin V-FITC and PI double labeling: quantitative detection of apoptosis

Quantitative detection of apoptosis rate is performed by the flow cytometry (Annexin V-FITC and PI double-labeling method)

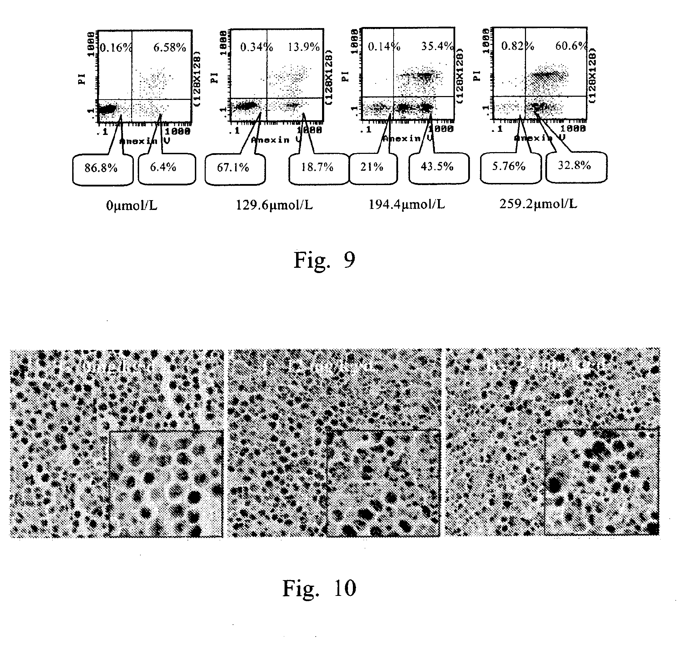

As shown in Fig. 9, in the scatter diagram, the lower left quadrant shows Annexin-V FITC and PI double-labeled negative cells (normal cells); the lower right quadrant shows Annexin-V FITC single-labeled positive cells (early apoptotic cells), the upper right quadrant shows Annexin-V FITC and PI double-labeled positive cells (late apoptotic cells), the upper left quadrant shows PI single- labeled positive cells (apoptotic cells).

The results indicate that, after tea polyphenols of varying concentrations acted on acute lymphoblastic leukemia primary culture cells for 1.5 days, in control group (0µmol/L) normal cells were 86.8%. After tea polyphenols of concentrations of 129.6µmol/L, 194.4µmol/L and 259.2µmol/L acting for 1.5 days, early apoptotic and late apoptotic cells increased obviously, while normal proliferating cells decreased significantly which are 67.1%, 21% and 5.76%, respectively.

Third, the therapeutic effects of tea polyphenols solution prepared by Tegreen97®, a green tea capsule, on tumor model in nude mice:

RPMI8226 cell line of 4 × 10⁸ cells per ml was prepared, and each mouse was injected 1.5 ml under back skin. Tumors appeared after about 10 days. From the 16th day after injection, the supernatant of capsule tea polyphenols solution was administered to the mice orally for 5 consecutive days, the concentration of EGCG was 48mg/ml and it was taken orally once per day. Physiological saline was administered to the control group. From the 16th day, the tumor sizes were measured every day. See results in the following table.

| Feeding dosage | Size of the lump (cm×cm) | | | | | | |
|---|---|---|---|---|---|---|---|
| | 16th days after injection | 17th day | 18th day | 19th day | 20th day | 21st day | 22nd day |
| 24mg/kg.d | 1.06×0.96 | 0.952×0.69 | 0.73×0.344 | 0.392×0.34 | 0.28×0.04 | | Dead |
| 12mg/kg.d | 0.786×0.756 | 0.76×0.70 | 0.68×0.54 | 0.55×0.52 | 0.38×0.22 | Dead | |
| Control group | 0.51×0.44 | 0.84×0.76 | Dead | | | | |

In the control group, the volume of tumor kept increasing after the 16th day and the mice died on the 18th day. In the treated group, since the medicament was administered from the 16th day, the tumor continually shrunk instead of growing and the dying time of mice was delayed for at least 3∼4 days in comparison with the control group. This indicates tea polyphenols have significant antitumor effects.

The tumor tissues of mice, which were fed with different dosages of tea polyphenols, were taken out, and then made into paraffin sections which were dyed by HE, and then the morphology change of the cells was observed by the microscope.

As shown in Fig. 10, the tumor tissue of mice which are fed with 0mg/kg·d tea polyphenols shows evenly lightly stained cell nuclei, loose chromatin and normal proportion of cytoplasmic; after the mice were fed with 12mg/kg·d and 24mg/kg·d tea polyphenols, membrane shrinkage, chromatin condensation, pyknotic nucleus and eosinophilic body (apoptosis body) appeared in tumor cells.

The results indicate that tea polyphenols can induce apoptosis of tumor cells in mice, and the higher the concentration is, the more obvious the effects are.

## Claims

1. Tea polyphenols or pharmaceutically acceptable salts thereof or pharmaceutical compositions containing them for use in the prevention or treatment of malignant tumors in a hematopoietic system, wherein the tea polyphenols comprise catechins which mainly consist of epigallocatechin gallate, epicatechin gallate and epigallocatechin, and in which the tea polyphenols, epigallocatechin gallate, epicatechin gallate and epigallocatechin are 50∼60 %, 20∼25 % and 8∼10 % by weight of the catechins, respectively.

2. The tea polyphenols or pharmaceutical compositions for use according to claim 1, **characterized in that** the tea polyphenols are tea leave extracts.

3. The tea polyphenols or pharmaceutical compositions for use according to claim 2, **characterized in that**, when the extraction is performed, the main active components, epigallocatechin gallate, epicatechin gallate and epigallocatechin in the tea polyphenols do not need to be further separated and purified, and can be used directly.

4. The tea polyphenols or pharmaceutical compositions for use according to claim 1 or 2, **characterized in that** the content of caffeine in the tea polyphenols is less than 0.2 % by weight.

5. The tea polyphenols or pharmaceutical compositions for use according to claim 4, **characterized in that**, when the extraction is performed, tannin in tea leaves is removed from the tea polyphenols.

6. The tea polyphenols or pharmaceutical compositions for use according to claim 1 or 5, **characterized in that** the total content of the catechins is 60∼70% by weight of the tea polyphenols.

7. The tea polyphenols or pharmaceutical compositions for use according to claim 6, **characterized in that**, in every 1000mg of formulations, there are 648 mg of the catechins, wherein epigallocatechin gallate, epicatechin gallate and epigallocatechin are 380mg, 148mg and 60mg, respectively.

8. The tea polyphenols or pharmaceutical compositions for use according to claim 1, **characterized in that** the tea polyphenols are Tegreen97®, a green tea capsule of Pharmanex INC. USA.

9. The tea polyphenols or pharmaceutical compositions for use according to claim 8, **characterized in that** the green tea capsule, Tegreen97®, is 250mg in weight and includes 162mg of the catechins, wherein epigallocatechin gallate, epicatechin gallate and epigallocatechin are 95mg, 37mg and 15mg, respectively.

10. The tea polyphenols or pharmaceutical compositions for use according to claim 1, further comprising pharmaceutically acceptable excipents and carriers.

11. The tea polyphenols or pharmaceutical compositions for use according to claim 1, **characterized in that** the tea polyphenols can be used alone.

12. The tea polyphenols or pharmaceutical compositions for use according to claim 1, formulated as tablets, capsule, buccal tablets, orally disintegrating tablets, oral fast dissolving tablets, dispersible tablet, masticatory, granules, dry suspension, injection, solution, slow-release formulation, controlled-release formulation or rapid-release formulation.

13. The tea polyphenols or pharmaceutical compositions for use according to claim 1, **characterized in that** the malignant tumors in the hematopoietic system comprise multiple myeloma RPMI8226 cells, Lymphoma such as Jurkat cells and Sudhl-4 cells, acute promyelocytic Leukemia such as NB4 cells, NB4-R1 cells and NB4-R2 cells, acute monocytic leukemia U937 cells, chronic myelocytic Leukemia K562 cells, primary culture cells of acute lymphoblastic leukemia and primary culture cells of acute monocytic leukemia.

## Patentansprüche

1. Tee-Polyphenole oder pharmazeutisch verträgliche Salze davon oder pharmazeutische Zusammensetzungen, welche diese enthalten, zur Verwendung bei der Vorbeugung oder Behandlung von bösartigen Tumoren in einem hämotopoetischen System, wobei die Tee-Polyphenole Catechine umfassen, die hauptsächlich aus Epigallocatechingallat, Epicatechingallat und Epigallocatechin bestehen und in welchen die Tee-Polyphenole Epigallocatechingallat, Epicatechingallat und Epigallocatechin 50-60 Gew.%, 20-25 Gew.% beziehungsweise 8-10 Gew.% der Catechine betragen.

2. Tee-Polyphenole oder pharmazeutische Zusammensetzungen zur Verwendung nach Anspruch 1, **dadurch gekennzeichnet, dass** die Tee-Polyphenole Teeblattextrakte sind.

3. Tee-Polyphenole oder pharmazeutische Zusammensetzungen zur Verwendung nach Anspruch 2, **dadurch gekennzeichnet, dass**, wenn die Extraktion durchgeführt wird, die hauptsächlichen aktiven Bestandteile Epigallocatechingallat, Epicatechingallat und Epigallocatechin in den Tee-Polyphenolen nicht weiter aufgetrennt und gereinigt werden müssen und direkt verwendet werden können.

4. Tee-Polyphenole oder pharmazeutische Zusammensetzungen zur Verwendung nach Anspruch 1 oder 2, **dadurch gekennzeichnet, dass** der Gehalt an Koffein in den Tee-Polyphenolen weniger als 0,2 Gew.% beträgt.

5. Tee-Polyphenole oder pharmazeutische Zusammensetzungen zur Verwendung nach Anspruch 4, **dadurch gekennzeichnet, dass**, wenn die Extraktion durchgeführt wird, Tannin in den Teeblättern aus den Tee-Polyphenolen entfernt wird.

6. Tee-Polyphenole oder pharmazeutische Zusammensetzungen zur Verwendung nach Anspruch 1 oder 5, **dadurch gekennzeichnet, dass** der Gesamtgehalt an Catechinen 60-70 Gew.% der Tee-Polyphenole beträgt.

7. Tee-Polyphenole oder pharmazeutische Zusammensetzungen zur Verwendung nach Anspruch 6, **dadurch gekennzeichnet, dass** in je 1000 mg der Formulierungen 648 mg Catechine vorkommen, wobei Epigallocatechingallat, Epicatechingallat und Epigallocatechin 380 mg, 148 mg beziehungsweise 60 mg betragen.

8. Tee-Polyphenole oder pharmazeutische Zusammensetzungen zur Verwendung nach Anspruch 1, **dadurch gekennzeichnet, dass** die Tee-Polyphenole Tegreen97® sind, eine Grünteekapsel von Pharmanex INC. USA.

9. Tee-Polyphenole oder pharmazeutische Zusammensetzungen zur Verwendung nach Anspruch 8, **dadurch gekennzeichnet, dass** die Grünteekapsel Tegreen97® ein Gewicht von 250 mg aufweist und 162 mg der Catechine umfasst, wobei Epigallocatechingallat, Epicatechingallat und Epigallocatechin 95 mg, 37 mg beziehungsweise 15 mg betragen.

10. Tee-Polyphenole oder pharmazeutische Zusammensetzungen zur Verwendung nach Anspruch 1, die ferner pharmazeutisch verträgliche Bindemittel und Trägerstoffe umfassen.

11. Tee-Polyphenole oder pharmazeutische Zusammensetzungen zur Verwendung nach Anspruch 1, **dadurch gekennzeichnet, dass** die Tee-Polyphenole allein verwendet werden können.

12. Tee-Polyphenole oder pharmazeutische Zusammensetzungen zur Verwendung nach Anspruch 1, formuliert als Tabletten, Kapseln, Buccaltabletten, im Mund zerfallende Tabletten, im Mund schnellauflösende Tabletten, dispergierbare Tabletten, Kautabletten, Granula, Trockensuspensionen, Injektionen, Lösungen, langsam freisetzende Formulierungen, kontrolliert freisetzende Formulierungen oder schnell freisetzende Formulierungen.

13. Tee-Polyphenole oder pharmazeutische Zusammensetzungen zur Verwendung nach Anspruch 1, **dadurch gekennzeichnet, dass** die bösartigen Tumoren in dem hämatopoetischen System multiple Myelom- RPM18226-Zellen, Lymphome wie etwa Jurkatzellen und Sudhl-4 Zellen, akute promyeloide Leukämie wie etwa NB4 Zellen, NB4-R1 Zellen und NB4-R2 Zellen, akute monozytische Leukämie-U937-Zellen, chronische myeloide Leukämie-K562-Zellen, primäre Kulturzellen von akuter lymphoblastischer Leukämie und primäre Kulturzellen von akuter monozytischer Leukämie umfassen.

## Revendications

1. Polyphénols de thé ou leurs sels acceptables au plan pharmaceutique ou compositions pharmaceutiques les contenant pour usage dans la prévention ou le traitement de tumeurs malignes dans un système hématopoïétique, dans lesquels les polyphénols de thé comprennent des catéchines qui sont principalement constituées de gallate d'épigallocatéchine, de gallate d'épicatéchine et d'épigallocatéchine et dans lesquels les polyphénols de thé, le gallate d'épigallocatéchine, le gallate d'épicatéchine et l'épigallocatéchine, constituent 50 à 60 %, 20 à 25 % et 8 à 10 % en poids des catéchines, respectivement.

2. Polyphénols de thé ou compositions pharmaceutiques pour usage selon la revendication 1, **caractérisés en ce que** les polyphénols de thé sont des extraits de feuilles de thé.

3. Polyphénols de thé ou compositions pharmaceutiques pour usage selon la revendication 2, **caractérisés en ce que**, lorsque l'extraction est effectuée, les composants actifs principaux, le gallate d'épigallocatéchine, le gallate d'épicatéchine et l'épigallocatéchine, dans les polyphénols de thé, n'ont pas besoin d'être encore séparés et purifiés et peuvent être utilisés directement.

4. Polyphénols de thé ou compositions pharmaceutiques pour usage selon la revendication 1 ou la revendication 2, **caractérisés en ce que** la teneur en caféine dans les polyphénols de thé est inférieure à 0,2 % en poids.

5. Polyphénols de thé ou compositions pharmaceutiques pour usage selon la revendication 4, **caractérisés en ce que**, lorsque l'extraction est effectuée, le tanin des feuilles de thé est éliminé des polyphénols de thé.

6. Polyphénols de thé ou compositions pharmaceutiques pour usage selon la revendication 1 ou la revendication 5, **caractérisés en ce que** la teneur totale en catéchines est de 60 à 70 % en poids des polyphénols de thé.

7. Polyphénols de thé ou compositions pharmaceutiques pour usage selon la revendication 6, **caractérisés en ce que**, dans chaque lot de 1000 mg de formulations, il y a 648 mg des catéchines, dans lesquels le gallate d'épigallocatéchine, le gallate d'épicatéchine et l'épigallocatéchine sont présents à raison de 380 mg, de 148 mg et de 60 mg, respectivement.

8. Polyphénols de thé ou compositions pharmaceutiques pour usage selon la revendication 1, **caractérisés en ce que** les polyphénols de thé sont formés de Tegreen97®, une capsule de thé vert de Pharmanex INC. USA.

9. Polyphénols de thé ou compositions pharmaceutiques pour usage selon la revendication 8, **caractérisés en ce que** la capsule de thé vert, le Tegreen97®, est présente à raison de 250 mg en poids et comprend 162 mg des catéchines, dans lesquels le gallate d'épigallocatéchine, le gallate d'épicatéchine et l'épigallocatéchine sont présents à raison de 95 mg, de 37 mg et de 15 mg, respectivement.

10. Polyphénols de thé ou compositions pharmaceutiques pour usage selon la revendication 1, comprenant en outre des excipients et des véhicules acceptables au plan pharmaceutique.

11. Polyphénols de thé ou compositions pharmaceutiques pour usage selon la revendication 1, **caractérisés en ce que** les polyphénols de thé peuvent être utilisés seuls.

12. Polyphénols de thé ou compositions pharmaceutiques pour usage selon la revendication 1, formulés sous la forme de comprimés, de capsules, de comprimés buccaux, de comprimés à désintégration orale, de comprimés à dissolution orale rapide, de comprimés dispersables, de gommes à mastiquer, de granulés, de suspension sèche, d'injection, de formulation à libération lente, de formulation à libération contrôlée ou de formulation à libération rapide.

13. Polyphénols de thé ou compositions pharmaceutiques pour usage selon la revendication 1, **caractérisés en ce que** les tumeurs malignes dans le système hématopoïétique comprennent des cellules RPMI8226 à myélomes multiples, des lymphomes tels que des cellules de Jurkat et des cellules Sudhl-4, des leucémies promyélocytaires aiguës telles que des cellules NB4, des cellules NB4-R1 et des cellules NB4-R2, des cellules U637 de leucémies monocytaires aiguës, des cellules K562 de leucémies myélocytaires chroniques, des cellules de culture primaire de leucémie lymphoblastique aiguë et des cellules de culture primaire de leucémie monocytaire aiguë.
